# EUROPEAN PATENT APPLICATION

(11) **EP 3 733 197 A1**
(43) Date of publication of application: **04.11.2020**
(21) Application number: 19172038.2
(22) Date of filing: 30.04.2019
(51) Int. Cl.: A61K 38/26, A61K 9/08, A61K 9/127

(54) **ANAEROBIC ANTIOXIDANT COMPOSITION**

(71) Applicant: Pitcher, Stephen N., Alpine, UT 84004 (US); Purser, Danny C., Provo, UT 84604 (US)
(72) Inventor: Pitcher, Stephen N., Alpine, UT 84004 (US); Purser, Danny C., Provo, UT 84604 (US)
(74) Representative: Forresters IP LLP

(57) **Abstract**

The disclosure relates to compositions, methods and systems for improving levels of free radicals in a body. A composition includes an antioxidant in a reduced state and a deoxygenated water solvent. The composition is such that the antioxidant remains in its reduced state.

## Description

### TECHNICAL FIELD

The disclosure relates generally to antioxidant compositions and particularly relates to anaerobic antioxidant compositions for improving free radical levels in a user.

### BACKGROUND

The disclosure relates to compositions, methods, and systems for improving free radical levels in a user and for the treatment of oxidative stress in a user. Oxidative stress is responsible for numerous conditions and diseases and can cause extensive damage to cells, proteins, and DNA (Deoxyribonucleic Acid). The compositions, methods, and systems disclosed herein mitigate oxidative stress and lower the levels of dangerous free radicals in a body.

Bodies are constantly under attack from oxidative stress. Oxygen molecules include two oxygen atoms that are bonded together with a covalent bond. These two bonded oxygen atoms can split into two separate oxygen atoms that each have one unpaired electron. Unpaired electrons are very unstable and will immediately seek out other unpaired electrons to form a stable pair of electrons. These unpaired electrons are referred to as "free radicals" and can be responsible for causing extensive damage to a body. When oxygen atoms have a free radical, *i.e.* an unpaired electron, the oxygen atom can cause oxidative stress in a body.

Free radicals are associated with numerous human diseases, including cancer, atherosclerosis, Alzheimer's disease, Parkinson's disease, and many others. Free radicals have a link to aging symptoms, and in fact aging has been defined as a gradual accumulation of free-radical damage. Substances that generate free radicals can be found in food, medicines, water, and air. Some substances that are known for having high quantities of free radicals include fried foods, alcohol, tobacco, pesticides, and air pollutants. Additionally, free radicals are the natural byproducts of chemical processes that take place in the body. Thus, the accumulation of free radicals in inevitable and it is important to mitigate the damage caused by free radicals and oxidative stress.

When a free radical is formed, a chain reaction can occur that can lead to broken cell membranes which can then alter what enters and exits a cell. The chain reaction may change the structure of a lipid, protein, or DNA strand. The damaged cells can mutate and turn into cancerous cells. The cascading damage initiated by a free radical can change DNA code that is then replicated by the body with the incorrect DNA sequence. Because free radicals can cause extensive damage to a body, it is desirable to reduce free radical levels in a body and thereby mitigate the damage caused by free radicals.

Oxidative stress occurs where there are too many free radicals and too much cellular damage caused by free radicals. Oxidative stress is associated with damage of proteins, lipids, and nucleic acids. Oxidative stress can play a role in the development of numerous conditions, including macular degeneration, cardiovascular disease, certain cancers, emphysema, alcoholism, Alzheimer's disease, Parkinson's disease, ulcers, inflammatory diseases, arthritis, lupus, and others. Some symptoms of oxidative stress include fatigue, headaches, noise sensitivity, memory loss and brain fog, muscle and joint pain, wrinkles, vision trouble, and decreased immunity. It is therefore desirable to reduce or eliminate oxidative stress in a body.

In light of the foregoing, the disclosure provides compositions, methods, and systems for improving free radical levels in a body and for treating oxidative stress. The compositions, methods, and systems of the disclosure are also applicable in the treatment and support of the body's natural detoxification process and/or the treatment or support of the body's natural defense systems for combatting viruses, bacteria, heavy metal toxicity, radiation, certain medications, the process of aging, and others.

### BRIEF DESCRIPTION OF THE DRAWINGS

Non-limiting and non-exhaustive implementations of the disclosure are described with reference to the following figures, wherein like reference numerals refer to like parts throughout the various views unless otherwise specified. Advantages of the disclosure will become better understood with respect to the following description and accompanying drawing where:
FIG. 1 illustrates the chemical structure of reduced glutathione (GSH) according to one implementation consistent with the teachings and principles of the disclosure;
FIG. 2 illustrates the chemical oxidation reaction of reduced glutathione (GSH) to oxidized glutathione (GSSG) according to one implementation consistent with the teachings and principles of the disclosure;
FIG. 3 illustrates a structure of a liposome and a lipid bilayer according to one implementation consistent with the teachings and principles of the disclosure;
FIG. 4 illustrates a standard curve for reduced glutathione resulting from a short-term analysis of a clinical example consistent with the teachings and principles of the disclosure;
FIG. 5 illustrates a standard curve for reduced glutathione resulting from a long-term analysis of a clinical example consistent with the teachings and principles of the disclosure;
FIG. 6 illustrates concentrations of reduced glutathione resulting from a short-term analysis of a clinical example consistent with the teachings and principles of the disclosure;
FIG. 7 illustrates concentrations of oxidized glutathione resulting from a short-term analysis of a clinical example consistent with the teachings and principles of the disclosure;
FIG. 8 illustrates ratios of reduced glutathione to oxidized glutathione resulting from a short-term analysis of a clinical example consistent with the teachings and principles of the disclosure;
FIG. 9 illustrates concentrations of reduced glutathione resulting from a long-term analysis of a clinical example consistent with the teachings and principles of the disclosure;
FIG. 10 illustrates concentrations of oxidized glutathione resulting from a long-term analysis of a clinical example consistent with the teachings and principles of the disclosure;
FIG. 11 illustrates ratios of reduced glutathione to oxidized glutathione resulting from a long-term analysis of a clinical example consistent with the teachings and principles of the disclosure;
FIG. 12 illustrates ratios of reduced glutathione to oxidized glutathione resulting from a long-term analysis versus the baseline analysis of a clinical example consistent with the teachings and principles of the disclosure;
FIG. 13 illustrates a schematic flow chart diagram of a method of improving free radical levels in a user consistent with the teachings and principles of the disclosure; and
FIG. 14 illustrates a schematic flow chart diagram of a method of improving free radical levels in a user consistent with the teachings and principles of the disclosure.

### DETAILED DESCRIPTION

Disclosed herein are compositions, methods, and systems for improving free radical levels in a body and/or for alleviating oxidative stress in a body. The compositions, methods, and systems disclosed herein can reduce and alleviate the damage caused by free radicals. An embodiment of the disclosure is a composition including an antioxidant and a deoxygenated water solvent. The composition is such that the antioxidant remains in a reduced state. The composition may be microencapsulated and may further be packaged in an airless container that is configured to maintain an anaerobic environment. The composition may be provided for topical, oral, intravenous, intramuscular administration, and/or inhalant administration.

An embodiment of the disclosure is a composition including reduced glutathione and a deoxygenated water solvent. The composition is such that the reduced glutathione remains in its reduced state and does not oxidize to form a more stable form of glutathione. The composition may be microencapsulated in a phospholipid liposomal structure or some other microencapsulation structure. The composition may be packaged in an airless dispenser that is configured to maintain an anaerobic environment.

The composition disclosed herein demonstrates unexpectedly good results for improving free radical levels in a body, alleviating oxidative stress in a body, and for treating or alleviating symptoms of multiple conditions and diseases. The composition disclosed herein demonstrates unexpectedly good results for treating burns, including alleviating pain associated with burns and for healing burns. The composition disclosed herein demonstrates unexpectedly good results for treating shingles and post herpetic neuralgia. The composition disclosed herein demonstrates unexpectedly good results for treating Herpes Type 1 and Type 2. The composition disclosed herein demonstrates unexpectedly good result for treating tardive dyskinesia. The composition disclosed herein demonstrates unexpectedly good results for treating or alleviating symptoms associated with the Epstein Barr Virus.

Free radicals can cause extensive damage to a body and persistent free radical damage can lead to oxidative stress. Free radicals are atoms or molecules that are highly reactive with other cellular structures. Free radicals include an unpaired electron that is highly unstable. Free radicals are natural byproducts of ongoing biochemical reactions in the body, including ordinary metabolic processes and immune system responses. Free radical-generating substances can be found in food, drugs, medicines, air, and water. Such substances include fried foods, alcohol, tobacco, pesticides, air pollutants, and many more. Free radicals can cause damage to parts of cells such as proteins, DNA, and cell membranes by stealing electrons through a process referred to as oxidation. The unpaired electron in a free radical is highly unstable and "steals" an electron from another molecule to perform a much more stable electron pair. When free radicals oxidize important components of a cell, those components lose the ability to function normally and the accumulation of such damage can cause a cell to die. Numerous studies indicate that increased production of free radicals causes or accelerates nerve cell injury and leads to disease.

Antioxidants, also known as "free radical scavengers," are compounds that either reduce the formation of free radicals or reach with and neutralize free radicals. Antioxidants often work by donating an electron to the free radical before the free radical can oxidize other cell components. Once the electrons of the free radical are paired, the free radical is stabilized and becomes nontoxic. Therapy aimed at increasing the availability of antioxidants in cells can be effective in preventing or slowing the course of diseases.

The compositions, methods, and systems disclosed herein provide unexpectedly effective means for reducing the levels of free radicals in a body. When the levels of free radicals in a body are reduced, oxidative stress and many other conditions can be treated or alleviated. The compositions, methods, and systems disclosed herein provide unexpectedly good results for maintaining reduced glutathione in a reduced state such that the reduced glutathione can serve as an effective scavenger of free radicals.

The disclosure further extends to the anaerobic manufacture, packaging, and delivery of therapeutic amounts of bio-effective reduced glutathione (GSH). In the following description of the disclosure, reference is made to the accompanying drawings, which form a part hereof, and in which is shown by way of illustration specific implementations in which the disclosure may be practiced. It is understood that other implementations may be utilized, and structural changes may be made without departing from the scope of the disclosure.

In describing and claiming the subject matter of the disclosure, the following terminology will be used in accordance with the definitions set out below.

As used herein, the terms "comprising," "including," "containing," "characterized by," and grammatical equivalents thereof are inclusive or open-ended terms that do not exclude additional, unrecited elements or method steps.

As used herein, the phrase "consisting of' and grammatical equivalents thereof exclude any element, step, or ingredient not specified in the claim.

As used herein, the phrase "consisting essentially of' and grammatical equivalents thereof limit the scope of a claim to the specified ingredients, materials or steps and those that do not materially affect the basic and novel characteristic or characteristics of the claimed disclosure.

As used herein, "effective amount" means an amount of an ingredient or a component of the product that is nontoxic, but sufficient to provide the desired effect and performance at a reasonable benefit/risk ratio attending any dietary supplement or product. For example, an effective amount of a vitamin or mineral is an amount sufficient to prevent a deficiency thereof and to reduce the incidence of some adverse effects.

Unless defined otherwise, all technical and scientific terms used herein have the same meanings as commonly understood by one of ordinary skill in the art to which this disclosure pertains and belongs.

Oxidative stress, or high levels of free radicals in the body, is responsible for numerous health issues and is virtually impossible to avoid. Oxidative stress reflects an imbalance between the systemic manifestation of reactive oxygen species and the body's ability to readily detoxify the reactive intermediates or repair the resulting damage. Disturbances in the body's normal redox state of cells can cause toxic effects through the production of peroxides and free radicals that damage all components of a cell, including proteins, lipids, and DNA. Oxidative stress from oxidative metabolism causes base damage as well as strand breaks in DNA. Further, some reactive oxidative species act as cellular messengers in redox signaling. Thus, oxidative stress may cause disruptions in the body's normal mechanisms of cellular signaling. In humans, oxidative stress is thought to be involved in the development of attention deficit hyperactivity disorder, cancer, Parkinson's disease, Lafora disease, Alzheimer's disease, atherosclerosis, heart failure, myocardial infarction, fragile X syndrome, sickle-cell disease, licen planus, vitiligo, autism, infection, chronic fatigue syndrome, and depression, among other.

Chemically, oxidative stress is associated with increased production of oxidizing species or a significant decrease in the effectiveness of antioxidant defenses, such as glutathione. Reduced glutathione (GSH) is often referred to as the body's master antioxidant. Glutathione is composed of three amino acids, including cysteine, glycine, and glutamine, and glutathione can be found in virtually every cell of the body. The highest concentration of glutathione is typically found in the liver where it serves a critical function in the body's natural detoxification process. This natural detoxification process is important for treating oxidative stress in the body and avoiding the production of damaging peroxides and free radicals.

Glutathione is an important component of the body's natural defense system. Glutathione can be depleted by compositions or processes that are associated with free-radical damage, including for example, viruses, bacteria, heavy metal toxicity, radiation, certain medications, and the process of aging. The depletion of glutathione is associated with lower immune function, increased vulnerability to infection, and a reduction in the liver's ability to detoxify the body. As the generation of free radicals exceeds the body's ability to neutralize and eliminate them, oxidative stress occurs. A primary function of glutathione is to alleviate oxidative stress.

Reduced glutathione is under tight homeostatic control both intracellularly and extracellularly. A dynamic balance is maintained between the synthesis of reduced glutathione, its recycling from oxidized glutathione, and its utilization.

Reduced glutathione is utilized as a cofactor by multiple peroxidase enzymes to detoxify peroxides that are generated by oxygen radical attack on biological molecules. Reduced glutathione is further utilized as a cofactor by transhydrogenases to reduce oxidized centers on DNA, proteins, and other biomolecules. Reduced glutathione is further utilized as a cofactor by glutathione S-transferases (hereinafter "GST") to conjugate reduced glutathione with endogenous substances (*e.g.* estrogens), exogenous electrophiles (*e.g.* arene oxides, unsaturated carbonyls, and organic halides), and diverse xenobiotics. Low GST activity may increase risk for disease, and paradoxically, some reduced glutathione conjugates can also be toxic.

Reduced glutathione can be depleted by direct attack by free radicals and other oxidative agents. The homeostatic glutathione redox cycle attempts to keep reduced glutathione repleted as it is being consumed. Amounts available from foods are limited (less than 150 mg/day), and oxidative depletion can outpace synthesis.

The liver is the largest reservoir of reduced glutathione. The parenchymal cells synthesize reduced glutathione for P450 conjugation and numerous other metabolic requirements, then export reduced glutathione as a systemic source of SH-reducing power. Reduced glutathione is carried in the bile to the intestinal luminal compartment. Epithelial tissues of the kidney tubules, intestinal lining, and lungs have substantial P450 activity and a modest capacity to export reduced glutathione.

Equivalents of reduced glutathione circulate in the blood predominately as cystine (*i.e.,* the oxidized and more stable form of cysteine.) Cells import cystine from the blood, reconvert it to cysteine, and form it to synthesize reduced glutathione. Conversely, inside the cell, reduced glutathione assists in re-reducing oxidized forms of other antioxidants such as ascorbate and alpha-tocopherol.

Reduced glutathione is an important cell protectant; it directly quenches reactive hydroxyl free radicals, other oxygen-centered free radicals, and radical centers on DNA and other biomolecules. Reduced glutathione is a primary protectant of the skin, lens, cornea, and retina against radiation damage and other biochemical foundations of P450 detoxification in the liver, kidneys, lungs, intestinal, epithelia, and other organs.

Reduced glutathione is the essential cofactor for many enzymes that require thiol-reducing equivalents, and it helps keep redox-sensitive active sites on enzymes in the necessary reduced state. Higher-order thiol cell systems, the metallothioneins, thioredoxins, and other redox regulator proteins are ultimately regulated by the levels of reduced glutathione, and the ration of reduced glutathione to oxidized glutathione. The balance of reduced and oxidized glutathione is crucial to homeostasis in the body, to stabilizing the cellular biomolecular spectrum, and to facilitating cellular performance and survival. Reduced glutathione and its metabolites interface with energetics and neurotransmitter syntheses through several prominent metabolic pathways. Reduced glutathione availability down-regulates the pro-inflammatory potential of leukotrienes and other eicosanoids. Recently discovered S-nitroso metabolites, generated in vivo from reduced glutathione and nitric oxide (NO), further diversify the impact of reduced glutathione on the body's metabolism.

According to the present disclosure, a reduced glutathione supplement may be manufactured in an anaerobic environment using one or more non-reactive gasses to ensure the maintenance of the reduced form, rather than the oxidized form, of glutathione. Additionally, de-oxygenated water may be used during the manufacturing process and as a carrier for the supplement. By using de-oxygenated water, very little of the oxygen is available in the water to convert the reduced glutathione to oxidized glutathione.

In an embodiment of the disclosure, a composition for reducing oxidative stress and/or improving free radical levels in a user is disclosed. The composition includes an effective amount of reduced glutathione for reducing oxidative stress in the user, and a deoxygenated water solvent. In an embodiment, the composition is encapsulated in an aqueous solution. In an embodiment, the composition is packaged in an airless dispenser configured to maintain an anaerobic environment.

In a further embodiment of the disclosure, a method of reducing oxidative stress in a user is disclosed. The method includes providing a composition to the user, wherein the composition comprises an effective amount of reduced glutathione for reducing oxidative stress in the user and a deoxygenated water solvent. In an embodiment, the composition is encapsulated in a phospholipid liposome structure and the composition is packaged in an airless dispenser configured to maintain an anaerobic environment.

In a further embodiment of the disclosure, a method for manufacturing a composition for reducing oxidative stress in a user is disclosed. The method includes mixing reduced glutathione powder and deoxygenated water in an anaerobic environment to form a reduced glutathione solution. The method includes packaging the reduced glutathione solution in an airless dispenser in an anaerobic environment such that the reduced glutathione is not substantially exposed to an oxygen source from manufacture to dispensing.

In an embodiment of the disclosure, reduced glutathione is dissolved in a deoxygenated water solvent. The deoxygenated water solvent has had dissolved oxygen (O₂) gasses removed from the water. Various techniques for removing dissolved oxygen from water are known in the art, including for example, boiling water at atmospheric pressure, boiling water at reduced pressure, purging water with nitrogen gas (N₂), and sonication of the water under reduced pressure. Additionally, water deoxygenation may be performed by bio reactive processes including, for example, yeast-based bio reactive processes. In an embodiment, highly pure deoxygenated water is utilized in combination with other components of the composition disclosed herein.

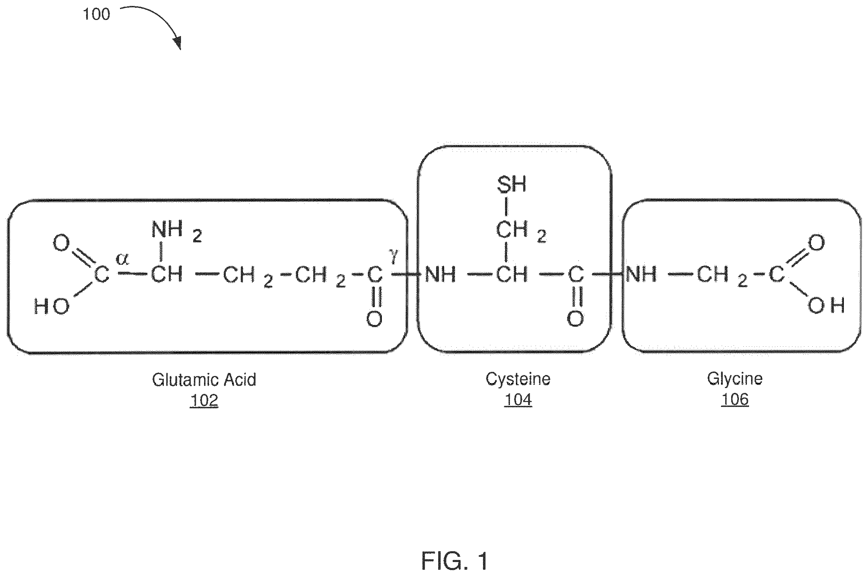

Referring now to the figures, FIG. 1 illustrates the chemical structure 100 of reduced glutathione. As illustrated, reduced glutathione comprises glutamic acid 102, cysteine 104, and glycine 106. Reduced glutathione (GSH) is a linear tripeptide of L-glutamine, L-cysteine, and glycine. Reduced glutathione is technically referred to as N-L-gamma-glutamyl-cysteinyl glycine or L-glutathione. The molecule includes a sulfhydryl (SH) group on the cysteinyl portion that accounts for its strong electron-donating character that enables glutathione to be effective in reducing oxidative stress in the body. Glutathione is oxidized as an electron is lost, and two such molecules (with a lost electron) become linked or dimerized by a disulfide bridge. The dimerized molecules form glutathione disulfide or oxidized glutathione (GSSG). The linkage is reversible upon re-reduction.

The synthesis of reduced glutathione includes two closely linked and enzymatically-controlled reactions that utilize adenosine triphosphate (ATP). First, cysteine and glutamate are combined by gamma-glutamyl cysteinyl synthetase. Second, reduced glutathione synthetase combines gamma-glutamylcysteine with glycine to generate reduced glutathione. As the levels of reduced glutathione rise, the processes are self-limited against further production of reduced glutathione. Otherwise, cysteine availability is usually rate-limiting. Fasting, protein-energy malnutrition, and other dietary amino acid deficiencies limit the synthesis of reduced glutathione. The recycling of reduced glutathione is catalyzed by glutathione disulfide reductase, which uses reducing equivalents from nicotinamide adenine dinucleotide phosphate (NADPH) to reconvert oxidized glutathione to glutathione disulfide. The reducing power of ascorbate helps conserve systemic reduced glutathione.

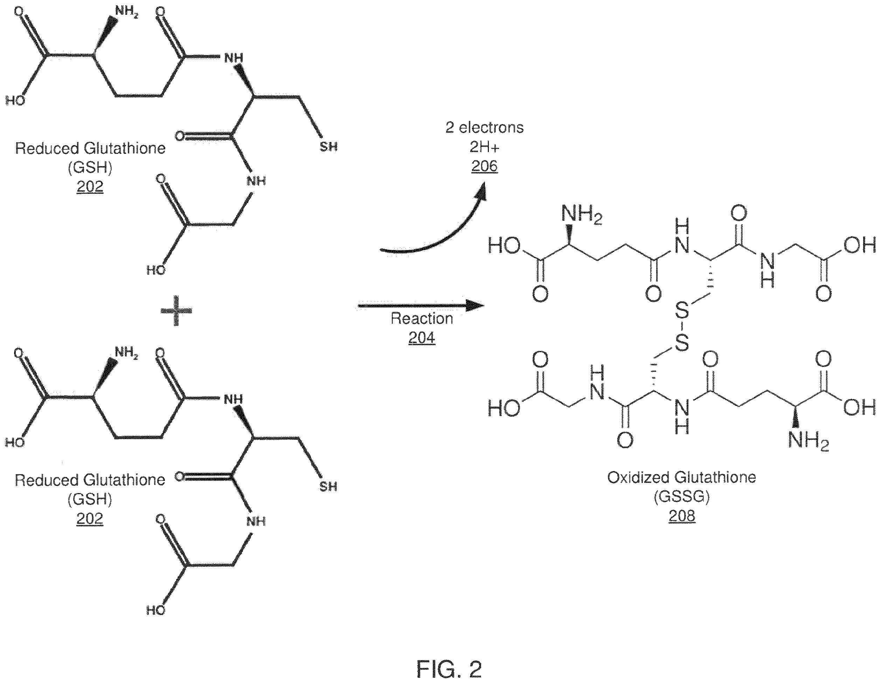

FIG. 2 illustrates the oxidation reaction 200 of reduced glutathione (GSH) 202 to oxidized glutathione (GSSG) 208. The reaction 200 produces two electrons and two protons as illustrated at 206.

In an embodiment of the disclosure, a composition is provided to a user. The composition includes 99.9% purity reduced glutathione powder. The reduced glutathione powder is solubilized in a deoxygenated water solvent and encapsulated in a plant-based phospholipid liposome structure. The composition includes an effective amount of stevia, natural lemon essential oil, and natural peppermint essential for improving the overall flavor of the composition for human oral consumption. In an embodiment, the composition includes from about 525 mg to about 575 mg of reduced glutathione per 4 g of solution.

In an embodiment, the resulting liquid composition is packaged in an airless dispenser that is configured to maintain the composition in an anaerobic environment. As such, the airless dispenser protects the reduced glutathione from degradation and conversion to oxidized glutathione. Applicant recognizes that previous purported supplements including reduced glutathione suffer from a sulfur smell due to the strong oxidation of reduced glutathione. As such, the airless dispenser extends the effective shelf-life of the reduced glutathione in the present disclosure by substantially eliminating contact of the reduced glutathione with oxygen, even after first use of the composition. Applicant recognizes that other forms of packaging and delivery result in the reduced glutathione making contact with oxygen and thus rapidly degrading and oxidizing into oxidized glutathione, even if a successful manufacture of reduced glutathione is initially achieved.

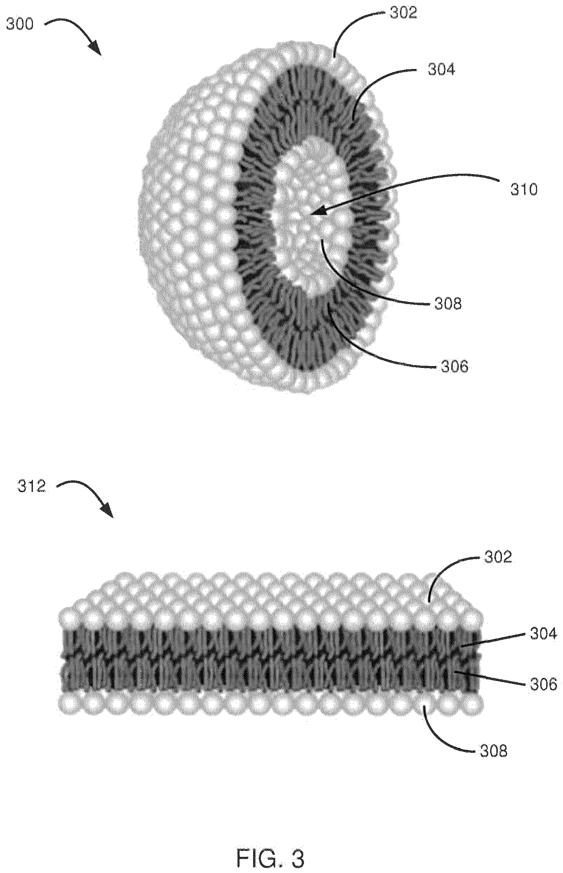

FIG. 3 illustrates a liposome structure 300 for microencapsulation of the composition of the disclosure. A composition including reduced glutathione and a deoxygenated water solvent may be microencapsulated in a liposome structure 300. In an embodiment, the reduced glutathione and deoxygenated water solvent are microencapsulated in a plant-based phospholipid liposome.

The liposome structure 300 includes a lipid bilayer 312. The lipid bilayer 312 is formed of two layers of lipids having opposite orientations. A lipid is a naturally occurring compound that is insoluble in polar solvents such as water. A lipid is insoluble in water due to a long hydrophobic hydrocarbon chain. The hydrophobic chain may be saturated or unsaturated. Lipids further include a glycerol molecule bonded to the long hydrophobic hydrocarbon chain. Depending on the type of lipid, the lipid may further include other molecules such as a phosphate group. The lipid bilayer 312 includes a first layer of lipids each having a first head group 302 and a first tail group 304. The lipid bilayer 312 includes a second layer of lipids each having a second head group 308 and a second tail group 306. As shown in the liposome structure 300 and the lipid bilayer 312, the first layer of lipids and the second layer of lipids are oriented in opposite directions. This orientation of the lipids is promoted by the natural water insolubility of the long hydrophobic hydrocarbon chains. When lipids are disposed in water, the lipids can form the liposome structure 300 because the long hydrophobic hydrocarbon chains are insoluble in water but the head groups are water-soluble. The water-soluble head groups can form a layer that is facing an aqueous solution both outside and inside the liposome structure 300. By contrast, the long hydrophobic hydrocarbon chains face into the middle of the lipid bilayer 312 and away from the aqueous solution located outside and inside the liposome structure 300.

The lipid bilayer 312 in the liposome structure 300 forms a core 310. Other molecules or substances can be disposed within the core 310 of the liposome structure 300. In an embodiment, the composition is disposed within the core 310. The reduced glutathione and/or deoxygenated water solvent may be disposed within the core 310 such that the reduced glutathione and/or deoxygenated water solvent is microencapsulated by the liposome structure 300.

In an embodiment, the composition includes reduced glutathione present in the composition from 5% to 20% by weight of the total composition. The composition further includes a deoxygenated water solvent. The composition is encapsulated by any suitable means. The composition may be encapsulated by way of "microencapsulation" with biopolymers, polysaccharides, hydrocolloids, or gums, and the composition may specifically be encapsulated in a liposome structure. Further, the composition may be encapsulated in a hard-shelled capsule and/or a soft-shelled capsule.

In an embodiment where the composition is encapsulated in a capsule, the capsule may be manufactured from an aqueous solution of gelling agents such as animal protein or plant polysaccharides or their derivatives such as carrageenans and modified forms of starch and cellulose. Other ingredients may be added to the capsules including plasticizers such as glycerin or sorbitol to decrease the capsule's hardness. Additionally, ingredients such as coloring agents, preservatives, disintegrants, lubricants, and/or surface treatments may be added. In an implementation, the composition is encapsulated in a soft-shelled capsule such as a single-piece gel encapsulation. A soft-shelled capsule can be an effective delivery system for oral drugs and especially for poorly soluble drugs. The soft-shelled capsule may include liquid ingredients that increase solubility or permeability of the composition. In an implementation, the composition is encapsulated in a hard-shelled capsule such as a two-piece gel encapsulation. The hard-shelled capsule can be an effective delivery system for oral drugs and especially for powdered drugs.

In an embodiment where the composition is encapsulated by way of microencapsulation, the reduced glutathione and/or deoxygenated water solvent is surrounded by a coating that promotes a slow release of the reduced glutathione into the body of the user. Microencapsulation is a process in which small particles or droplets are surrounded by a coating to produce a small capsule. In the present disclosure, microencapsulation may be employed to enclose reduced glutathione inside a micrometric wall made of a hard of soft soluble film. The microencapsulation of reduced glutathione can promote the slow release of reduced glutathione and prevent the degradation of oxidation of the highly unstable reduced glutathione. A microcapsule is a small sphere with a uniform or mostly uniform wall around it, and the wall may be referred to as a shell, coating, membrane, or capsule. The microcapsule may have a diameters between a few micrometers and a few millimeters. The wall of the microcapsule may be formed of lipids or polymers. Example coating materials for the wall of the microcapsule include ethyl cellulose, polyvinyl alcohol, gelatin, and sodium alginate. In various embodiments, the composition is microencapsulated by way of pan coating, centrifugal extrusion, vibrational nozzle, spray-drying, ionotropic gelation, coacervation-phase separation, interfacial polycondensation, interfacial cross-linking, in situ polymerization, and/or matrix polymerization.

The composition may be microencapsulated with various different materials. The composition may be microencapsulated with a plant-based material. Some plant-based carbohydrate polymers that are suitable for microencapsulation include starch, polysaccharide, cellulose, plant exudates such as gum Arabic, um karaya, and mesquite gum, and plant extracts such as galactomannans and soluble soybean. Some plant-based proteins that are suitable for microencapsulation include gluten and isolates such as pea or soy. Some plant-based lipids that are suitable for microencapsulation include fatty acids, alcohols, glycerides, waxes, and phospholipids. The composition may be microencapsulated with a marine-based material. Some marine-based carbohydrate polymers suitable for microencapsulation include carrageenan and alginate. The composition may be microencapsulated with a microbial or animal-based material. Some microbial or animal-based carbohydrate polymers that are suitable for microencapsulation include xanthan, gellan, dextran, and chitosan. Some microbial or animal-based proteins that are suitable for microencapsulation include caseins, whey proteins, and gelatin. Some microbial or animal-based lipids that are suitable for microencapsulation include fatty acids, alcohols, glycerides, waxes, and phospholipids.

In an embodiment, the composition is microencapsulated with a lipid. Lipids exhibit general insolubility in water and include molecules and substances of large diversity and structural variety such as oils, fats, waxes, and phospholipids. The composition may particularly be microencapsulated with a phospholipid. Phospholipids include two long chain fatty acids and a hydroxyl group. Numerous species of phospholipids are possible by variation of the different head groups and fatty acyl substitution at the first and second position of the glycerol backbone. Phospholipids may form a liposome structure that encapsulates the reduced glutathione and/or deoxygenated water solvent of the present disclosure. The liposome structure forms an aqueous interior that is separated by one or more phospholipid bilayers from the aqueous exterior. The manufacturing techniques used, and the intensity of mixing employed will govern the size of the liposome structure.

The microencapsulated composition that includes reduced glutathione and deoxygenated water solvent shows unexpectedly good results for maintaining reduced glutathione in the highly unstable reduced form. When the reduced glutathione remains reduced, the reduced glutathione remains effective for scavenging free radicals in the user's body and thereby "neutralizing" the free radicals or rendering them non-toxic in the body. Further, the microencapsulated composition demonstrates unexpectedly good results for promoting the slow release of reduced glutathione into the body such that the reduced glutathione is used effectively and is not immediately oxidized.

### Clinical Example Testing The Bio-Effectiveness Of The Composition

In one study conducted to test the effectiveness of a composition comprising reduced glutathione as disclosed above, the bio-effectiveness and availability of reduced glutathione in the body was greatly enhanced compared with purported GSH supplements known in the art. The composition provided unexpectedly good results given that purported GSH supplements known in the art in fact fail to deliver reduced glutathione to the user.

In one clinical study, a composition including reduced glutathione in a deoxygenated water solvent was provided to a user. The composition was encapsulated in a phospholipid liposome structure and packaged and delivered from an airless dispenser. The composition included 550±2 mg of reduced glutathione per 4 g of solution. The composition was provided to three human volunteers as a liquid-based oral supplement, and it was provided to one human volunteer as a liquid-based topical treatment to be applied topically. The human serum levels of reduced glutathione and oxidized glutathione were sampled initially before ingestion to establish a baseline level, and the serum levels were again sampled three times over eight hours after ingestion or application of the composition. The participants continued to take the composition once every morning at least thirty minutes before meals for four weeks. The serum levels of the participants were sampled once each week during the four-week study period to collect data for long-term effects of the composition.

Each participant experienced a short-term increase in serum levels of reduced glutathione equal to at least a 30% increase in the presence of reduced glutathione in the serum. Each participant experienced a long-term progressive reduction of oxidized glutathione equal to at least a 30% reduction in the presence of oxidized glutathione in the serum. As discussed previously, the ratio of reduced glutathione to oxidized glutathione is an important and well-understood marker for cellular oxidative stress. The results of the clinical example indicate that the composition was bioavailable unlike other pill and capsule delivery forms of purported GSH supplements. Further, the composition provided long-term reduction in systemic cellular oxidative stress in each of the participants.

In the above-referenced study, four persons agreed to participate. The participants included one male and one female ranging in age from 23 to 83 years of age. The participants were assigned a number from #1 to #4 as follows: (1) 61-year-old female, (2) 55-year-old male, (3) 23-year-old female, and (4) 83-year-old female. Each of the participants was permitted to each the morning of the first serum collection but refrained from taking antioxidant dietary supplements during the course of the four-week study period. The participants' blood was collected for baseline measurements at approximately 8:00 AM by intravenous puncture. The serum was extracted, processed, and frozen for later analysis. After the collection of the baseline serum sample, the participants immediately consumed the composition by dispensing 4 mL of the liquid composition into their mouth and swishing for 15-20 seconds before swallowing. The participants refrained from drinking for 15 minutes afterward. The participant who used the product topically applied approximately the same amount of the composition onto the abdomen and soft areas under the arms. Samples of the participants' blood were against collected at 2 hours, 6 hours, and 8 hours after the first collection. The participants consumed the composition one time per day in the morning for a four-week period. The participants' blood was collected each week at exactly seven-day intervals for four weeks at approximately 10:00 AM. The participants were instructed to consume the composition four hours prior to collection. The serum was extracted, processed, and frozen for later analysis. All serum samples were assayed within thirty days of collection.

### Analysis of the Serum Samples.

The serum samples were analyzed to determine the levels of reduced glutathione and oxidized glutathione. The serum samples were analyzed using the BioVision (Milpitas, CA) Glutathione Fluorometric Assay Kit (GSH, GSSG, and Total) k264. Wasatch Scientific Laboratories in Murray, Utah, USA was contracted to perform the assay using the BioVision fluorometric kit and method. Whole blood samples were collected and immediately centrifuged to separate the serum from the red blood cells and heavier components. Approximately 120 µL of serum was added to 40 µL of an ice-cold perchloric acid PCA buffer in a 1 mL aliquot. The solution was vortexed and stored on ice for five minutes. The solution was centrifuged at 13,000 G for two minutes, and the supernatant was collected and frozen at -60degC.

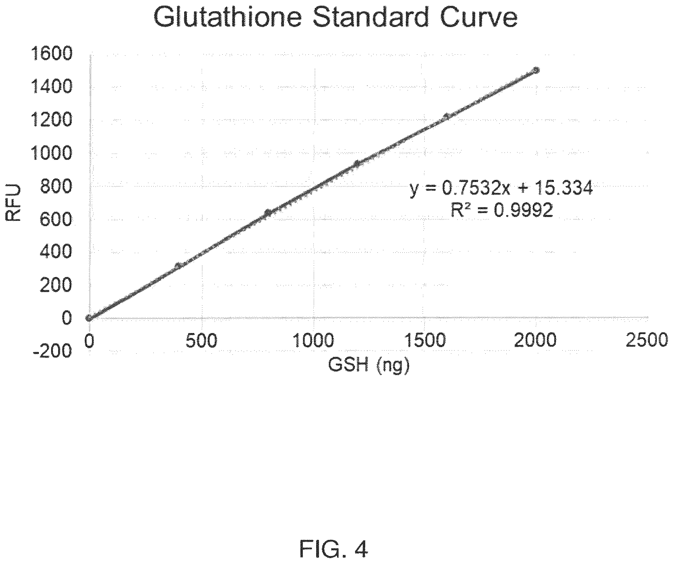

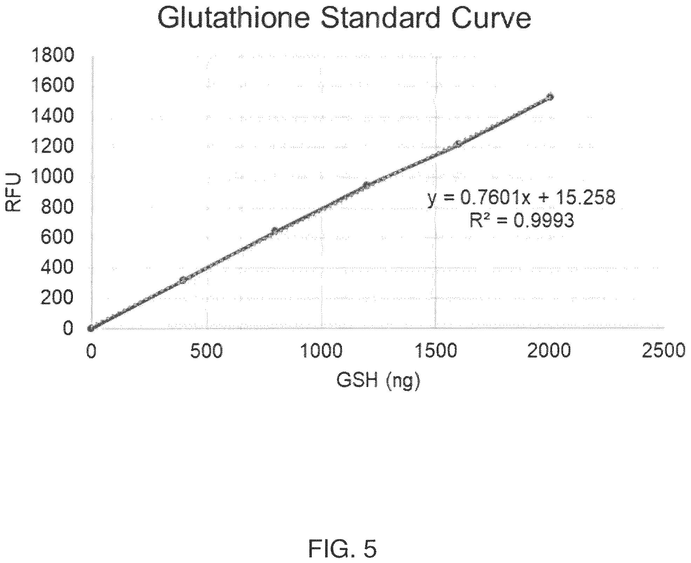

FIG. 5 illustrates a standard curve for reduced glutathione for the short-term analysis. The standard curve for reduced glutathione and oxidized glutathione were created, and then the prepared serum samples were tested at two dilutions to determine the optimal dilution for the best dynamic resolution of the assay. The samples were processed and assayed in duplicate pairs at the chosen dilution. The results of each pair were reviewed and compared for repeatability and best-fit samples were used. FIG. 4 illustrates a mass of reduced glutathione (GSH) in nanograms on the x-axis plotted against the relative fluorescence units (RFU) on the y-axis.

FIG. 5 illustrates a standard curve for reduced glutathione for the long-term analysis. The standard curve for reduced glutathione and oxidized glutathione were created, and then the prepared serum samples were tested at two dilutions to determine the optimal dilution for the best dynamic resolution of the assay. The samples were processed and assayed in duplicate pairs at the chosen dilution. The results of each pair were reviewed and compared for repeatability and best-fit samples were used. FIG. 4 illustrates a mass of reduced glutathione (GSH) in nanograms on the x-axis plotted against the relative fluorescence units (RFU) on the y-axis. The results of the assay were compiled and displayed in tabular and graphical form. In addition, ratiometric results of reduced glutathione (GSH) and oxidized glutathione (GSSG) were created for each sample.

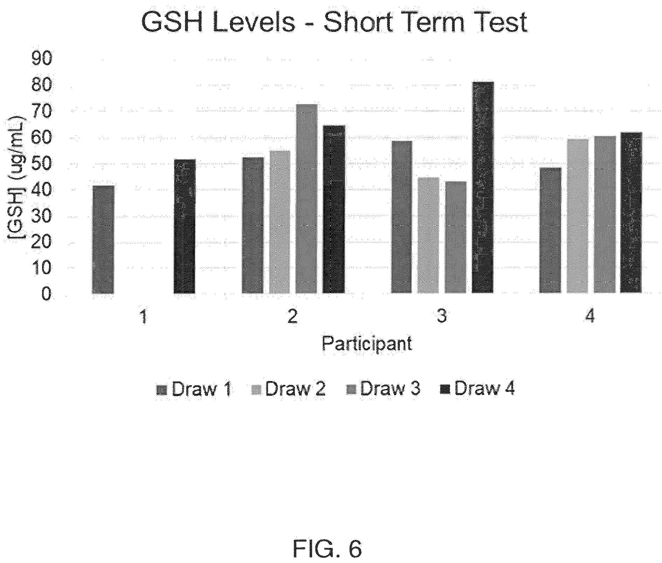

FIG. 6 illustrates concentrations of reduced glutathione for each of the four aforementioned participants for the short-term analysis. FIG. 6 illustrates concentrations in ***µg*/*mL*** for each of four possible serum draws for each of the four participants. Draw one is the baseline draw. Draw two was taken at the baseline plus four hours. Draw three was taken at the baseline plus six hours. Draw four was taken at the baseline plus eight hours. As illustrated in FIG. 6, all participants in the short-term analysis experienced an increase in serum levels of reduced glutathione. The increase concentration of reduced glutathione appeared to peak at approximately six to eight hours after ingestion. The increase in reduced glutathione was on average 30% above baseline levels. Participant one, a female, applied the composition topically and experienced elevated serum levels of reduced glutathione.

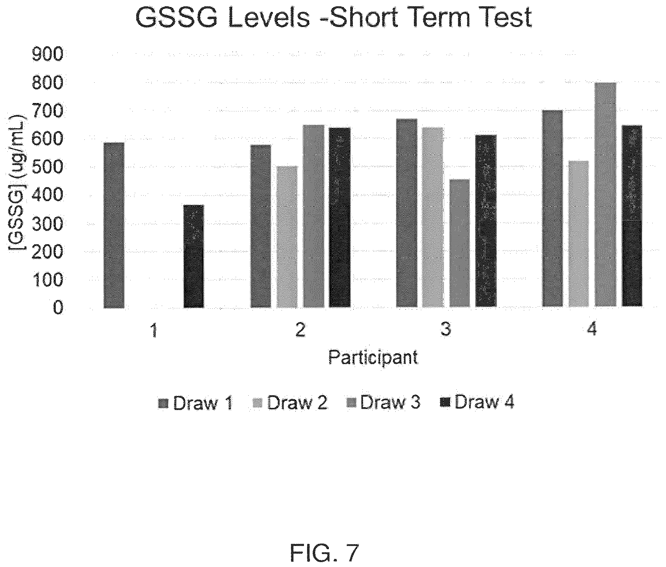

FIG. 7 illustrates concentrations of oxidized glutathione for each of the four aforementioned participants for the short-term analysis. FIG. 7 illustrates concentrations in ***µg*/*mL*** for each of four possible serum draws for each of the four participants. Draw one is the baseline draw. Draw two was taken at the baseline plus four hours. Draw three was taken at the baseline plus six hours. Draw four was taken at the baseline plus eight hours. Participant one, a female, applied the composition topically and experienced elevated serum levels of reduced glutathione. As illustrated in FIG. 7, concentrations of oxidized glutathione appeared to respond differently for each participant. The most significant change in levels of oxidized glutathione was an almost 50% reduction in participant one who applied the composition topically. In participants three and four, the levels of oxidized glutathione on average reduced slightly from baseline.

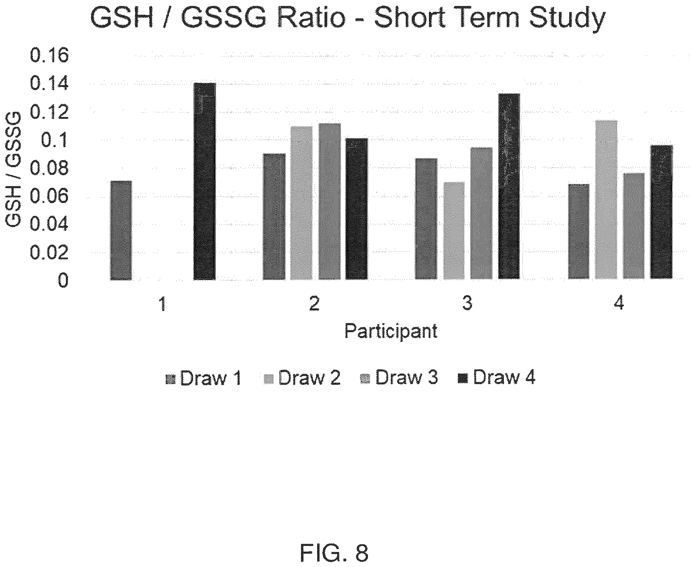

FIG. 8 illustrates the ratios of reduced glutathione to oxidized glutathione for each of the participants for the short-term analysis. As illustrated in FIG. 8, the composition provided a short-term antioxidant benefit to each of the participants. The reduced/oxidized glutathione ratio is an accepted standard to measure cellular oxidative stress. This study illustrates the redox ratio trend over time. After ingestion, each participant experienced an increase in the redox ration compared to the baseline, even eight hours after consuming or applying the composition.

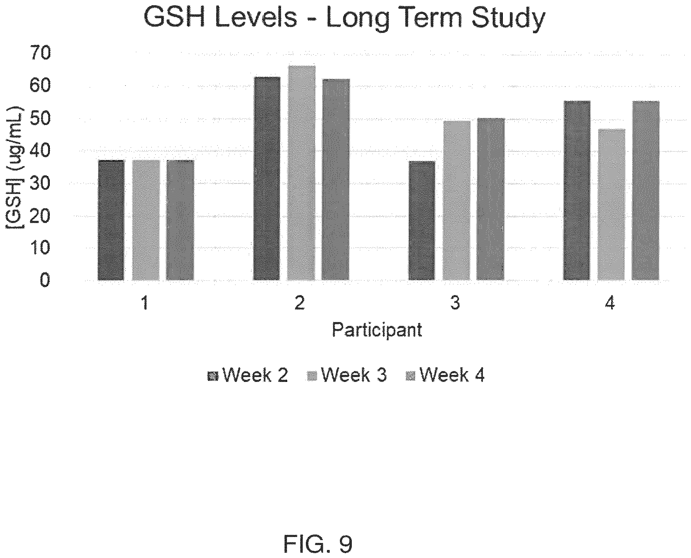

FIG. 9 illustrates concentrations of reduced glutathione for each of the four aforementioned participants for the long-term analysis. FIG. 9 illustrates concentrations in ***µg*/*mL*** for each of three weekly serum draws for each of the four participants. Draw one (not shown due to laboratory error) was taken at 10:00 AM exactly one week after ingestion or application of the composition. Draw two occurred at 10:00 AM two weeks after ingestion or application of the composition. Draw three occurred at 10:00 AM exactly three weeks after ingestion or application of the composition. Draw four occurred at 10:00 AM exactly four weeks after ingestion or application of the composition. The human serum samples were assayed for concentrations of reduced and oxidized glutathione. Participant one, a female, applied the composition topically and participants two, three, and four ingested orally a liquid-based composition.

As illustrated in FIG. 9, the participants' serum levels of reduced glutathione show mixed results. Participants one and three demonstrates levels of reduced glutathione that are very close to the baseline levels without significant increase. Participants two and four demonstrated long-term increase in levels of reduced glutathione equal to approximately 20-22%. The long-term serum levels of reduced glutathione when viewed alone showed positive results in participants two and four.

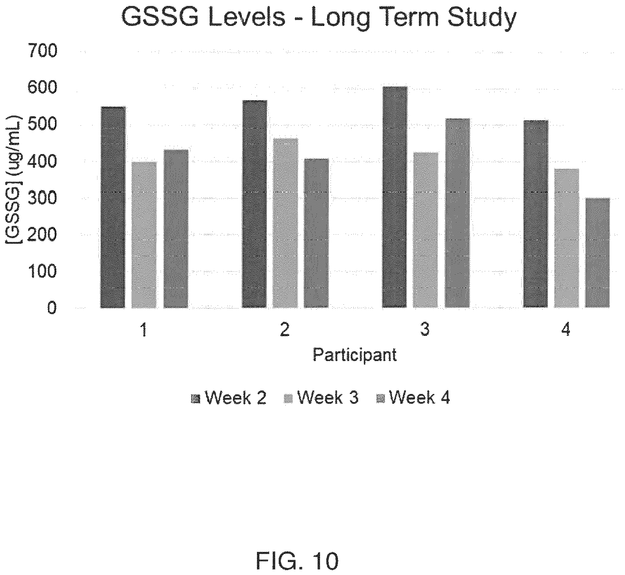

FIG. 10 illustrates concentrations of oxidized glutathione for each of the four aforementioned participants for the long-term analysis. FIG. 10 illustrates concentrations in ***µg*/*mL*** for each of three weekly serum draws for each of the four participants. Draw one (not shown due to laboratory error) was taken at 10:00 AM exactly one week after ingestion or application of the composition. Draw two occurred at 10:00 AM two weeks after ingestion or application of the composition. Draw three occurred at 10:00 AM exactly three weeks after ingestion or application of the composition. Draw four occurred at 10:00 AM exactly four weeks after ingestion or application of the composition. The human serum samples were assayed for concentrations of reduced and oxidized glutathione. Participant one, a female, applied the composition topically and participants two, three, and four ingested orally a liquid-based composition.

As illustrated in FIG. 10, each of the four participants experienced a significant decrease in long-term concentrations of oxidized glutathione (GSSG). As discussed previously, reduced glutathione is oxidized inside cells and converted to oxidized glutathione according to the reaction 200 illustrated in FIG. 2. A portion of the oxidized glutathione is shuttled out of the cells and into the intracellular space and into the blood. When the ratio of reduced/oxidized glutathione is calculated, a true reduction is cellular oxidation is evidence.

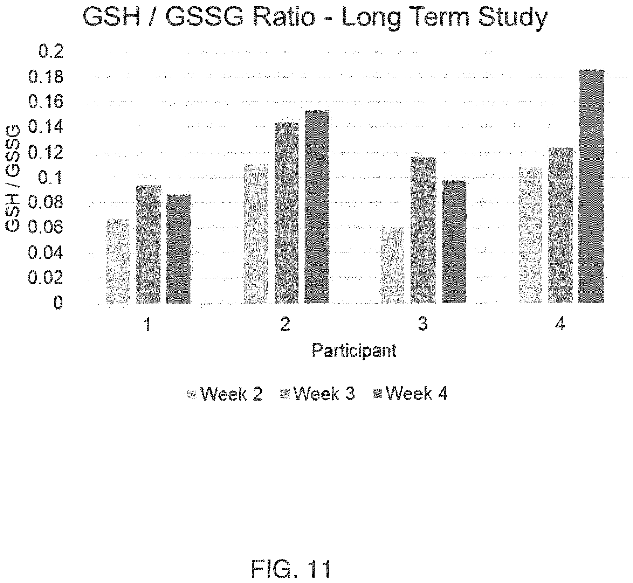

FIG. 11 illustrates the ratios of reduced glutathione to oxidized glutathione for each of the four aforementioned participants for the long-term analysis. As illustrated in FIG. 11, each of the participants experienced a sustained and cumulative decrease in oxidative stress. The reduced/oxidized glutathione redox ratio increased for all participants over the four weeks of ingesting or applying the composition. Applicant notes that participant three and participant four represent a wide age and health gap, where one is a 23-year-old healthy female and the other is an 83-year old female with health issues due to age. The composition had a positive long-term effect in both the young participant and the older participant, where the older participant experienced a greater rise in the redox ratio. The data further shows that participant one experienced a redox benefit, even by applying the product topically. Applicant notes that participant one only applied about one-third of the dose compared to the oral participants, not following specific dosing instructions. The results for all participants clearly demonstrates the benefits of the novel composition comprising reduced glutathione that is disclosed herein.

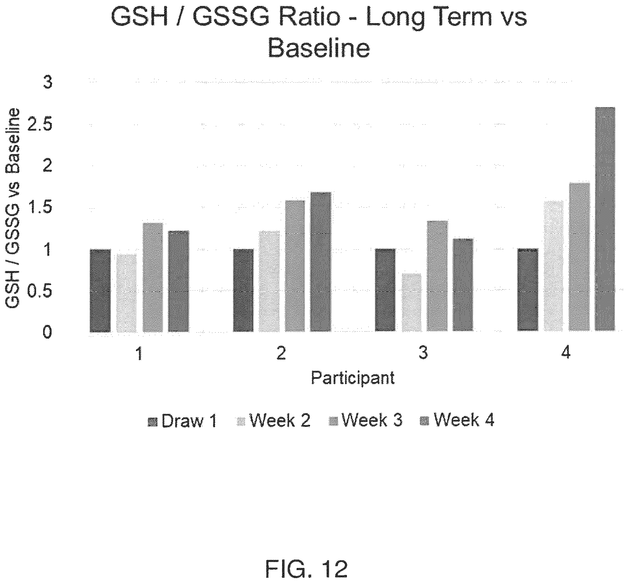

FIG. 12 illustrates the ratios of reduced glutathione to oxidized glutathione for each of the participants for the long-term analysis versus the baseline analysis. FIG. 12 illustrates a comparison of the change in redox over time as a result of supplementation with the composition disclosed herein. As illustrated in FIG. 12, all participants experienced a net average increase above baseline redox ratios, with participant two (55-year-old male) and participant four (83-year-old female) experiencing the greatest increase. Applicant notes that it is anticipated that participant one (61-year-old female) would have experienced a greater result if the dosage requirements for topical application were followed more consistently.

The effects of the novel composition comprising reduced glutathione that is disclosed herein indicate a bioavailability of reduced glutathione and its short-term and long-term benefits in the human body as indicated in the serum concentrations of reduced glutathione (GSH) and oxidized glutathione (GSSG). All participants experienced a noticeable increase in GSH/GSSG redox ratio indicating a true reduction in cellular oxidative stress, with the older participants (age 55-83) experiencing higher ratios. This is consistent with the assumption that older participants have a greater amount of cellular oxidative stress to be reduced.

The results of the clinical example demonstrate conclusively that the composition disclosed herein is effectively delivering reduced glutathione. The composition includes reduced glutathione and a deoxygenated water solvent. The composition is manufactured and packaged in an anaerobic environment, in stark contrast with previous supplements known in the art that purport to deliver reduced glutathione but instead deliver oxidized glutathione. The careful maintenance of an anaerobic condition, as disclosed herein, provides the unexpected result (given all prior failures) of a composition that genuinely comprises reduced glutathione. The composition disclosed herein provides increased levels of reduced glutathione, reduced cellular oxidative stress, and provides all the appurtenant benefits.

It should be appreciated that in various embodiments, dosage amounts may be adjusted depending on the effect on any given individual, as well as the biometrics of that individual. For example, a larger person may require a higher dosage than a smaller person. Similarly, a person suffering from an acute condition may benefit from a higher dose for a time until the condition is controlled, at which point a lower dose may provide sufficient management over time.

### Clinical Example For Treatment Of Skin Burns

In one embodiment, the composition is provided for the treatment of burns and for burn pain relief and healing. In a study, the composition was applied topically to greater than 200 patients experiencing severe pain caused by first, second, or third degree burns. The patients reported complete pain resolution within minutes of topical application of the composition. Additionally, the patients reported rapid healing of the burn.

### Clinical Example For Treatment Of Shingles And Postherpetic Neuralgia

In one embodiment, the composition is provided for the treatment of shingles and postherpetic neuralgia (PHN). In a study, the composition is provided to 103 patients for the treatment of shingles and/or PHN. Each of the greater than 103 patients participating in the study was over the age of 70 years and experienced active shingles lesions and neuralgia for at least three years. The composition was applied topically to each of the 103 patients. Of the 103 patients, 98% reported pain resolution within 34 minutes. The remaining 2% reported pain resolution within two days. In the study, there was zero incidence of Post Herpatic Neuralgia after the composition was topically applied.

### Clinical Example For The Treatment Of Herpes Type 1 And Type 2

In one embodiment, the composition is provided for the treatment of Herpes Type 1 and/or Type 2. In a study, the composition is topically applied to patients with Type 1 or Type 2 Herpetic lesions. Each of the patients reported pain relief and resolution of herpetic symptoms. In the study, for patients that experienced a reoccurrence of a herpetic lesion, the composition was reapplied and the patient experienced quick resolution of the pain and other symptoms.

In a study, the composition is topically applied for the treatment of cold stores. The study includes 12 patients that have experienced cold sores and report the sensation that a cold sore lesion will appear. In the study, the composition is topically applied to the area where the patient reports the cold sore lesion will appear. For each of the 12 patients, the cold sore lesion never blossomed and never appeared.

### Clinical Example For The Treatment Of Tardive Dyskinesia

In one embodiment, the composition is provided for the treatment of tardive dyskinesia. In a study, the composition is topically applied over the course of six weeks. In the study, the composition is topically applied to two patients. Each of the two patients experienced complete resolution of the tardive dyskinesia. In the study, each of the two patients had been wheelchair and bed bound for at least ten years. Upon receiving topical application of the composition over the course of six weeks, each of the two patients was able to walk, eat, drink, and converse. The patients admitted to having been aware of all they had experienced over the course of the ten years they suffered from tardive dyskinesia.

### Clinical Example For The Treatment Of Diabetic Neuropathy

In one embodiment, the composition is provided for the treatment of diabetic neuropathy. In a study, the composition is topically applied to greater than one-hundred patients. In the study, approximately 5 mL of the composition is rubbed topically on the foot and/or leg of each of the greater than one-hundred patients. In the study, the patients reported experiencing complete pain relief after about four hours. In the study, the patients reported complete permanent relief after receiving treatment for 3-6 months.

### Clinical Example For The Treatment Of Symptoms Associated With The Epstein Barr Virus

In one embodiment, the composition is provided for the treatment of symptoms associated with the Epstein Barr Virus (EBV). In a study, the composition is provided for oral consumption to 25 patients. In the study, each of the 25 patients reported that symptoms resolved in 3-5 days after the oral dose of the composition. In the study, the composition was provided to the patients at a dosage of approximately 1100 mg per day.

In one embodiment, the composition may be provided for the treatment of cellular oxidative stress. When the composition is provided for the treatment of cellular oxidative stress, it may be ingested orally or administered topically. Applicant notes that in further embodiments the composition may be administered intravenously or intramuscularly, or in a tablet or powder form without departing from the scope of the disclosure.

In one embodiment, the composition is provided for the treatment of acute levels of free radicals in the body. In such an embodiment, the composition may be prepared for immediate intravenous or intramuscular administration. The composition may further or alternatively be provided for oral ingestion or topical application. In such an embodiment, the composition may be provided for regular administration after acute levels of free radicals in the body have been treated and reduced to safe levels.

In one embodiment, the composition is provided for the treatment of acetaminophen overdose. The molecule known as N-acetylcysteine (hereinafter "NAC") is known in the art for the treatment of acetaminophen overdose. Pharmaceutical NAC is primarily used in medical settings for respiratory conditions, to manage acetaminophen overdose, and to prevent radio-contrast-induced nephropathy. However, the half-life of NAC is approximately 5.6 hours, and 30% of NAC is renally excreted by the body. Orally administered or inhaled NAC is associated with drowsiness, stomatitis, clamminess, rhinorrhea, and hemoptysis, and NAC is a category B pregnancy risk. In contrast with NAC, where the half-life is extremely short and the molecule is unstable, the shelf-life of the composition disclosed herein is at least one year. Therefore, the composition disclosed herein provides an alternative to NAC that is shelf-stable for an extended period and demonstrates high viability and efficacy.

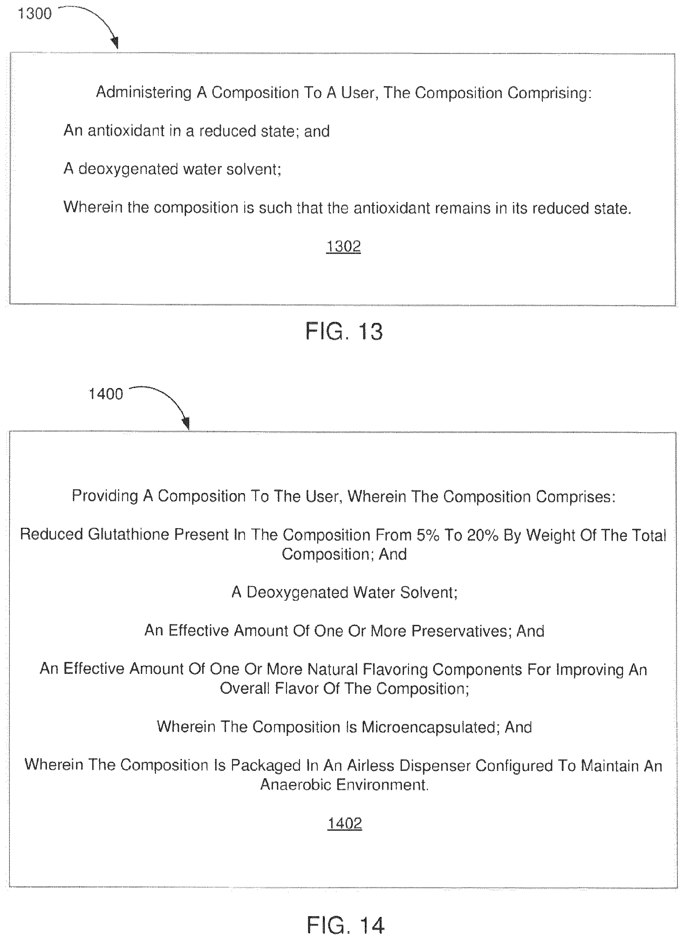

Referring now to FIG. 13, a schematic block diagram of a method 1300 for improving free radical levels in a user is illustrated. The method 1300 begins and a composition is provided to a user at 1302. The composition includes an antioxidant in a reduced state and a deoxygenated water solvent. The composition is such that the antioxidant remains in its reduced state.

FIG. 14 is a schematic block diagram of a method 1400 for improving free radical levels in a user. The method 1400 begins and a composition is provided to a user at 1402. The composition includes reduced glutathione present in the composition from 5% to 20% by weight of the total composition. The composition includes a deoxygenated water solvent. The composition includes an effective amount of one or more preservatives. The composition includes an effective amount of one or more natural flavoring components for improving an overall flavor of the composition. The composition is microencapsulated. The composition is packaged in an airless dispenser configured to maintain an anaerobic environment.

In one embodiment, a 99.9% purity reduced glutathione powder is solubilized in deoxygenated water and encapsulated in a plant-based phospholipid liposome structure. In the embodiment, the composition includes from about 8% to about 14% by weight reduced glutathione. The composition includes from about 4% to about 11% by weight sunflower lecithin. The composition includes from about 0.2% to about 0.6% by weight lemon essential oil. The composition includes from about 0.1% to about 0.5% peppermint essential oil. The composition includes from about 0.01% to about 0.25% stevia extract. The composition includes sodium benzoate and potassium sorbate.

In an embodiment of the disclosure, the composition includes highly pure L-glutathione reduced. In an embodiment of the disclosure, the reduced glutathione comprises a purity from about 98% to about 99.9% purity. In an embodiment, the purity is from about 90% to about 99.9% purity. In an embodiment, the purity is from about 95% to about 99.9% purity. In an embodiment, the purity is from about 99.0% to about 99.9% purity. It should be appreciated that various ranges of purity may be extracted from any of the aforementioned ranges as if those ranges were disclosed explicitly.

In an embodiment of the disclosure, the composition includes sunflower lecithin. Lecithin is a fat found in a plurality of food products. Sunflower lecithin is a phospholipid comprising phosphatidylcholine, phosphatidylinositol, phosphatidylethanolamine, and omega-6 linoleic acid. Sunflower lecithin is associated with processing fats and supporting cell membranes. In an embodiment of the disclosure, the sunflower lecithin is provided for encapsulating the composition and protecting the reduced glutathione component.

In an embodiment of the disclosure, the composition includes lemon essential oil. Lemon essential oil may be provided to improve an overall flavor of the composition. As disclosed, oxidized glutathione provides a sulfur smell that may be pungent or off-putting to a user. Additionally, the composition of the present disclosure may oxidize with oxygen in the air when the composition is being ingested, and the oxidized may cause the composition to become unpalatable to the user immediately before the user ingests the composition. The lemon essential oil may be provided to counteract the sulfur smell of the oxidized glutathione and increase the flavor of the composition.

In an embodiment of the disclosure, the composition includes peppermint essential oil. As discussed previously with respect to the lemon essential oil, the peppermint essential oil may be provided to improve the overall flavor of the composition and mask a sulfur smell arising from the oxidation of the reduced glutathione.

In an embodiment of the disclosure, the composition includes stevia extract. As discussed previously with respect to the lemon essential oil and/or the peppermint essential oil, the stevia extract may be provided to improve the overall flavor of the composition and mask a sulfur smell arising from the oxidation of the reduced glutathione.

In an embodiment of the disclosure, the composition includes sodium benzoate. Sodium has a chemical formula of NaC₇H₅O₂. Sodium benzoate is known as a food preservative and has an E number of E211. Sodium benzoate is the sodium salt of benzoic acid and exists in the form when dissolved in water. As a food additive, sodium benzoate is bacteriostatic and fungistatic under acidic condition. Sodium benzoate is included in the composition as a preservative in an embodiment of the disclosure.

In an embodiment of the disclosure, the composition includes potassium sorbate. Potassium sorbate has a chemical formula of CH₃CH=CH-CH=CH-CO₂K. Potassium sorbate is a known food preservative and has an E number of E202. Potassium sorbate is known for inhibiting molds and yeasts in a variety of foods and liquids. Potassium sorbate is included in the composition as a preservative in an embodiment of the disclosure.

### Examples

Chart 1 below shows an example embodiment of the composition. Components were dissolved in deoxygenated water and encapsulated in a plant-based phospholipid liposome structure. The composition was packaged and stored in an airless dispenser.

**Chart 1:**

| Component | Weight Percent Total Composition |
|---|---|
| L-glutathione reduced | 14.00 |
| Sunflower lecithin | 10.50 |
| Lemon essential oil | 0.40 |
| Peppermint essential oil | 0.25 |
| Sodium benzoate | 0.10 |
| Potassium sorbate | 0.10 |
| Stevia extract | 0.10 |

Chart 2 below shows an example embodiment of the composition. Components were dissolved in deoxygenated water and encapsulated in a plant-based phospholipid liposome structure. The composition was packaged and stored in an airless dispenser.

**Chart 2:**

| Component | Weight Percent Total Composition |
|---|---|
| L-glutathione reduced | 10.00 |
| Sunflower lecithin | 10.50 |
| Lemon essential oil | 0.40 |
| Peppermint essential oil | 0.25 |
| Sodium benzoate | 0.10 |
| Potassium sorbate | 0.10 |
| Stevia extract | 0.10 |

Chart 3 below shows an example embodiment of the composition. Components were dissolved in deoxygenated water and encapsulated in a plant-based phospholipid liposome structure. The composition was packaged and stored in an airless dispenser.

**Chart 3:**

| Component | Weight Percent Total Composition |
|---|---|
| L-glutathione reduced | 8.00 |
| Sunflower lecithin | 10.50 |
| Lemon essential oil | 0.40 |
| Peppermint essential oil | 0.25 |
| Sodium benzoate | 0.10 |
| Potassium sorbate | 0.10 |
| Stevia extract | 0.10 |

Chart 4 below shows an example embodiment of the composition. Components were dissolved in deoxygenated water and encapsulated in a plant-based phospholipid liposome structure. The composition was packaged and stored in an airless dispenser.

**Chart 4:**

| Component | Weight Percent Total Composition |
|---|---|
| L-glutathione reduced | 14.00 |
| Sunflower lecithin | 4.00 |
| Lemon essential oil | 0.40 |
| Peppermint essential oil | 0.25 |
| Sodium benzoate | 0.10 |
| Potassium sorbate | 0.10 |
| Stevia extract | 0.10 |

Chart 5 below shows an example embodiment of the composition. Components were dissolved in deoxygenated water and encapsulated in a plant-based phospholipid liposome structure. The composition was packaged and stored in an airless dispenser.

**Chart 5:**

| Component | Weight Percent Total Composition |
|---|---|
| L-glutathione reduced | 14.00 |
| Sunflower lecithin | 11.00 |
| Lemon essential oil | 0.40 |
| Peppermint essential oil | 0.25 |
| Sodium benzoate | 0.10 |
| Potassium sorbate | 0.10 |
| Stevia extract | 0.10 |

Chart 6 below shows an example embodiment of the composition. Components were dissolved in deoxygenated water and encapsulated in a plant-based phospholipid liposome structure. The composition was packaged and stored in an airless dispenser.

**Chart 6:**

| Component | Weight Percent Total Composition |
|---|---|
| L-glutathione reduced | 14.00 |
| Sunflower lecithin | 10.50 |
| Lemon essential oil | 0.20 |
| Peppermint essential oil | 0.25 |
| Sodium benzoate | 0.10 |
| Potassium sorbate | 0.10 |
| Stevia extract | 0.10 |

Chart 7 below shows an example embodiment of the composition. Components were dissolved in deoxygenated water and encapsulated in a plant-based phospholipid liposome structure. The composition was packaged and stored in an airless dispenser.

**Chart 7:**

| Component | Weight Percent Total Composition |
|---|---|
| L-glutathione reduced | 14.00 |
| Sunflower lecithin | 10.50 |
| Lemon essential oil | 0.60 |
| Peppermint essential oil | 0.25 |
| Sodium benzoate | 0.10 |
| Potassium sorbate | 0.10 |
| Stevia extract | 0.10 |

Chart 8 below shows an example embodiment of the composition. Components were dissolved in deoxygenated water and encapsulated in a plant-based phospholipid liposome structure. The composition was packaged and stored in an airless dispenser.

**Chart 8:**

| Component | Weight Percent Total Composition |
|---|---|
| L-glutathione reduced | 14.00 |
| Sunflower lecithin | 10.50 |
| Lemon essential oil | 0.40 |
| Peppermint essential oil | 0.10 |
| Sodium benzoate | 0.10 |
| Potassium sorbate | 0.10 |
| Stevia extract | 0.10 |

Chart 9 below shows an example embodiment of the composition. Components were dissolved in deoxygenated water and encapsulated in a plant-based phospholipid liposome structure. The composition was packaged and stored in an airless dispenser.

**Chart 9:**

| Component | Weight Percent Total Composition |
|---|---|
| L-glutathione reduced | 14.00 |
| Sunflower lecithin | 10.50 |
| Lemon essential oil | 0.40 |
| Peppermint essential oil | 0.50 |
| Sodium benzoate | 0.10 |
| Potassium sorbate | 0.10 |
| Stevia extract | 0.10 |

Example 1 is a composition for reducing oxidative stress in a user. The composition includes an effective amount of reduced glutathione for reducing oxidative stress in the user, and deoxygenated water solvent.
Example 2 is a composition as in Example 1, wherein the composition is encapsulated in a phospholipid liposome structure.
Example 3 is a composition as in any of Examples 1-2, wherein the composition is packaged in an airless dispenser configured to maintain an anaerobic environment.
Example 4 is a composition as in any of Examples 1-3, wherein the reduced glutathione comprises from about 8% to about 14% by weight of the total composition.
Example 5 is a composition as in any of Examples 1-4, wherein the composition comprises from about 500 mg to about 600 mg reduced glutathione per 4 g of the composition.
Example 6 is a composition as in any of Examples 1-5, wherein the reduced glutathione comprises a purity from about 98% to about 99.9% purity.
Example 7 is a composition as in any of Examples 1-6, further comprising an effective amount of sodium benzoate for preserving the composition.
Example 8 is a composition as in any of Examples 1-7, further comprising an effective amount of potassium sorbate for preserving the composition.
Example 9 is a composition as in any of Examples 1-8, further comprising an effective amount of natural flavoring comprising one or more of: lemon essential oil, peppermint essential oil, and stevia extract.
Example 10 is a composition as in any of Examples 1-9, wherein the composition is prepared for liquid oral consumption.
Example 11 is a composition as in any of Examples 1-10, wherein the composition is prepared for topical application.
Example 12 is a composition as in any of Examples 1-11, wherein the composition is prepared for intravenous or intramuscular administration.
Example 13 is a composition as in any of Examples 1-12, wherein the reduced glutathione comprises from about 4 g to about 20 g per 100 mL of the deoxygenated water solvent.
Example 14 is a method of reducing oxidative stress in a user. The method includes providing a composition to the user, wherein the composition includes an effective amount of reduced glutathione for reducing oxidative stress in the user, and a deoxygenated water solvent.
Example 15 is a method as in Example 14, wherein the composition is encapsulated in a phospholipid liposome structure.
Example 16 is a method as in any of Example 14-15, wherein the composition is packaged in an airless dispenser configured to maintain an anaerobic environment.
Example 17 is a method as in any of Example 14-16, wherein the reduced glutathione comprises from about 8% to about 14% by weight of the total composition.
Example 18 is a method as in any of Example 14-17, wherein the composition comprises from about 500 mg to about 600 mg reduced glutathione per 4 g of the composition.
Example 19 is a method as in any of Example 14-18, wherein the reduced glutathione comprises a purity from about 98% to about 99.9% purity.
Example 20 is a method as in any of Example 14-19, wherein the reduced glutathione comprises a purity from about 99.0% to about 99.9% purity.
Example 21 is a method as in any of Example 14-20, wherein the composition further comprises an effective amount of sodium benzoate for preserving the composition.
Example 22 is a method as in any of Example 14-21, wherein the composition further comprises an effective amount of potassium sorbate for preserving the composition.
Example 23 is a method as in any of Example 14-22, wherein the composition further comprises an effective amount of natural flavoring for improving an overall flavor of the composition, wherein the natural flavoring comprises one or more of: lemon essential oil, peppermint essential oil, and stevia extract.
Example 24 is a method as in any of Example 14-23, wherein the composition is provided to the user for liquid oral consumption.
Example 25 is a method as in any of Example 14-24, wherein the composition is provided to the user for topical administration.
Example 26 is a method as in any of Example 14-25, wherein the composition is provided to the user for intravenous or intramuscular administration.
Example 27 is a method as in any of Example 14-26, wherein the reduced glutathione comprises from about 4 g to about 20 g per 100 mL of the deoxygenated water solvent.
Example 28 is a method as in any of Example 14-27, wherein the composition is provided to the user for short-term treatment of cellular oxidative stress.
Example 29 is a method as in any of Example 14-28, wherein the composition increases blood-serum levels of reduced glutathione in the user by at least 25%.
Example 30 is a method as in any of Example 14-29, wherein the composition is provided for long-term treatment of cellular oxidative stress.
Example 31 is a composition. The composition includes an antioxidant in a reduced state and a deoxygenated water solvent. The composition is such that the antioxidant remains in its reduced state.
Example 32 is a composition as in Example 31, wherein the composition is microencapsulated.
Example 33 is a composition as in any of Examples 31-32, wherein the composition is packaged in an airless dispenser configured to maintain an anaerobic environment.
Example 34 is a composition as in any of Examples 31-33, wherein the antioxidant is reduced glutathione and wherein the reduced glutathione comprises from 8% to 14% by weight of the total composition.
Example 35 is a composition as in any of Examples 31-34, wherein the composition is microencapsulated in a liposome structure.
Example 36 is a composition as in any of Examples 31-35, wherein the composition is microencapsulated in a gelatin composition.
Example 37 is a composition as in any of Examples 31-36, wherein the antioxidant is reduced glutathione, and wherein the reduced glutathione comprises a purity from 98% to 99.9% purity in its dry state.
Example 38 is a composition as in any of Examples 31-37, further comprising an effective amount of one or more preservatives selected from a list comprising: potassium sorbate, sodium benzoate, sorbic acid, benzoic acid, natural benzyl alcohol, erythorbic acid, sodium erythorbate, ferrous gluconate, methyl paraben, potassium benzoate, rosemary extract, and sodium citrate.
Example 39 is a composition as in any of Examples 31-38, further comprising an effective amount of one or more natural flavoring components for improving an overall flavor of the composition selected from a list comprising: lemon essential oil, peppermint essential oil, monk fruit extract, agave, honey, natural cane sugar, glucose, fruit concentrate, natural fruit powder, spearmint essential oil, wintergreen essential oil, orange essential oil, tangerine essential oil, lavender essential oil, and stevia extract.
Example 40 is a composition as in any of Examples 31-39, wherein the antioxidant is reduced glutathione and wherein the composition comprises from 4 g to 20 g reduced glutathione per 100 mL of the deoxygenated water solvent.
Example 41 is a composition as in any of Examples 31-40, wherein the composition is provided for reducing oxidative stress in a user.
Example 42 is a composition as in any of Examples 31-41, wherein the composition is provided for improving free radical levels in a user.
Example 43 is a composition as in any of Examples 31-42, wherein the composition is provided for the treatment of skin burns.
Example 44 is a composition as in any of Examples 31-43, wherein the composition is provided for treatment of shingles.
Example 45 is a composition as in any of Examples 31-44, wherein the composition is provided for treatment of Herpes Type 1 and/or Herpes Type 2.
Example 46 is a composition as in any of Examples 31-45, wherein the composition is provided for treatment of tardive dyskinesia.
Example 47 is a composition as in any of Examples 31-46, wherein the composition is provided for treatment of diabetic neuropathy.
Example 48 is a composition as in any of Examples 31-47, wherein the composition is provided for treatment of symptoms associated with Epstein Barr Virus.
Example 49 is a composition as in any of Examples 31-58, wherein the composition is prepared for administration with an inhaler.
Example 50 is a composition as in any of Examples 31-59, wherein the composition is prepared for topical administration.
Example 51 is a composition as in any of Examples 31-50, wherein the composition is prepared for intravenous administration.
Example 52 is a composition as in any of Examples 31-51, wherein the composition is prepared for intramuscular or subcutaneous administration.
Example 53 is a composition as in any of Examples 31-52, wherein the composition is prepared for liquid oral consumption.
Example 54 is a composition as in any of Examples 31-53, wherein the antioxidant is reduced glutathione.

According to one or more embodiments of the disclosure, a composition may include a combination of all or some, but not all, of the following ingredients:
(a) L-glutathione reduced;
(b) deoxygenated water;
(c) sunflower lecithin;
(d) lemon essential oil;
(e) peppermint essential oil;
(f) sodium benzoate;
(g) potassium sorbate; and/or
(h) stevia extract.

Other embodiments of the composition may comprise, for example, concentrations of L-glutathione reduced as follows:
(a1) from 5% to 20% by weight the total composition;
(a2) from 6% to 19% by weight the total composition;
(a3) from 7% to 18% by weight the total composition;
(a4) from 8% to 17% by weight the total composition;
(a5) from 8% to 16% by weight the total composition;
(a6) from 8% to 15% by weight the total composition;
(a7) from 8% to 14% by weight the total composition;
(a8) from 8% to 13% by weight the total composition;
(a9) from 9% to 14% by weight the total composition;
(a10) from 10% to 14% by weight the total composition;
(a11) from 9% to 13% by weight the total composition;
(a12) from 10% to 12% by weight the total composition;
(a13) from 10% to 11% by weight the total composition.

With respect to ingredient (c) noted above for example, the amount of sunflower lecithin that may be included in the final composition is based on a percent by weight of the total weight of the final composition described herein. The composition may comprise ingredient (c) for example, in concentrations as follows:
(c1) from 1% to 20% by weight the total composition;
(c2) from 2% to 19% by weight the total composition;
(c3) from 3% to 18% by weight the total composition;
(c4) from 4% to 17% by weight the total composition;
(c5) from 4% to 16% by weight the total composition;
(c6) from 4% to 15% by weight the total composition;
(c7) from 4% to 14% by weight the total composition;
(c8) from 4% to 13% by weight the total composition;
(c9) from 4% to 12% by weight the total composition;
(c10) from 4% to 11% by weight the total composition;
(c11) from 5% to 10% by weight the total composition;
(c12) from 6% to 9% by weight the total composition;
(c13) from 6% to 8% by weight the total composition.

The foregoing percentages, concentrations, and ratios are presented by example only and are not intended to be exhaustive or to limit the disclosure to the precise percentages, concentrations, and ratios disclosed. It should be appreciated that each value that falls within a disclosed range is disclosed as if it were individually disclosed as set forth herein. For example, a range indicating a weight percent from about 8% to about 14% additionally includes ranges beginning or ending with all values within that range, including for example a range beginning at 8.1%, 8.2%, 8.3%, and so forth.

Also, according to one or more non-limiting embodiments of the disclosure, any of the concentrations for ingredients (a) or (c), for example, as listed above, may indicate the concentration for any of ingredients (b) and (d) thru (h), as listed above. For example, an embodiment of the disclosure may comprise, for example, (a7) from 8% to 14% by weigh the total composition of L-glutathione reduced, and equal parts by weight of lemon essential oil and peppermint essential oil. For example, the composition may comprise all, or any combination of but not all, of the ingredients (a) thru (h).

The foregoing description has been presented for the purposes of illustration and description. It is not intended to be exhaustive or to limit the disclosure to the precise form disclosed. Many modifications and variations are possible in light of the above teaching. Further, it should be noted that any or all the aforementioned alternate implementations might be used in any combination desired to form additional hybrid implementations of the disclosure.

Further, although specific implementations of the disclosure have been described and illustrated, the disclosure is not to be limited to the specific forms or arrangements of parts so described and illustrated. The scope of the disclosure is to be defined by the claims appended hereto, any future claims submitted here and in different applications, and their equivalents.

## Claims

1. A composition comprising:
an antioxidant in a reduced state; and
a deoxygenated water solvent;
wherein the composition is such that the antioxidant remains in its reduced state.

2. The composition of claim 1, wherein the antioxidant is reduced glutathione.

3. The composition of claim 1, wherein the composition is microencapsulated.

4. The composition of claim 1, wherein the composition is packaged in an airless dispenser configured to maintain an anerobic environment.

5. The composition of claim 1, wherein the antioxidant comprises from 5% to 20% by weight of the total composition.

6. The composition of claim 1, wherein the composition is microencapsulated in a phospholipid liposome structure.

7. The composition of claim 1, wherein the composition is microencapsulated in a gelatin composition.

8. The composition of claim 1, wherein the antioxidant is reduced glutathione and wherein the composition comprises from 4 g to 20 g reduced glutathione per 100 mL of the deoxygenated water solvent.

9. The composition of claim 1, wherein the antioxidant is reduced glutathione dissolved in the deoxygenated water solvent, and wherein the reduced glutathione comprises a purity from 98% to 99.9% purity in a dry state.

10. The composition of claim 1, wherein the composition is prepared for liquid oral consumption.

11. The composition of claim 1, wherein the composition is prepared for topical application.

12. The composition of claim 1, wherein the composition is prepared for intravenous or intramuscular administration.

13. The composition of claim 1, wherein the composition is prepared for inhalant delivery.

14. A method comprising:
administering a composition to a user, the composition comprising:
an antioxidant in a reduced state; and
a deoxygenated water solvent;
wherein the composition is such that the antioxidant remains in its reduced state.

15. The method of claim 14, wherein the antioxidant is reduced glutathione.

16. The method of claim 14, wherein the composition is microencapsulated.

17. The method of claim 14, wherein the composition is packaged in an airless dispenser configured to maintain an anerobic environment.

18. The method of claim 14, wherein the antioxidant comprises from 5% to 20% by weight of the total composition.

19. The method of claim 14, wherein the composition is microencapsulated in a phospholipid liposome structure.

20. The method of claim 14, wherein the composition is microencapsulated in a gelatin composition.

21. The method of claim 14, wherein the antioxidant is reduced glutathione and wherein the composition comprises from 4 g to 20 g reduced glutathione per 100 mL of the deoxygenated water solvent.

22. The method of claim 14, wherein the antioxidant is reduced glutathione dissolved in the deoxygenated water solvent, and wherein the reduced glutathione comprises a purity from 98% to 99.9% purity in a dry state.

23. The method of claim 14, wherein the composition is administered by way of liquid oral consumption.

24. The method of claim 14, wherein the composition is administered by way of topical application.

25. The method of claim 14, wherein the composition is administered by way of intravenous or intramuscular administration.

26. The method of claim 14, wherein the composition is administered with an inhaler.
